# FASCICULE DE BREVET EUROPEEN

(11) **EP 3 551 247 B1**
(45) Date de publication et mention de la délivrance du brevet: **14.10.2020**
(21) Numéro de dépôt: 17808107.1
(22) Date de dépôt: 20.11.2017
(51) Int. Cl.: A61M 1/10, A61M 1/12

(54) **ENSEMBLE POUR LA POSE/DÉPOSE D'UNE POMPE CARDIAQUE**
ANORDNUNG ZUR MONTAGE/ENTFERNUNG EINER HERZPUMPE
ASSEMBLY FOR FITTING/REMOVING A HEART PUMP

(30) Priorité: 09.12.2016 FR 1662266
(43) Date de publication de la demande: 16.10.2019
(73) Titulaire: Fineheart, 33600 Pessac (FR)
(72) Inventeur: GARRIGUE, Stéphane, 33130 Begles (FR); MASCARELL, Arnaud, 37250 Montbazon (FR)
(74) Mandataire: Plasseraud IP
(86) Numéro de dépôt international: PCT/FR2017/053182
(87) Numéro de publication internationale: WO 2018/104606

(56) Documents cités:
- US-A1- 2009 093 764
- US-A1- 2015 258 260
- US-A1- 2016 175 501

## Description

### ARRIERE-PLAN DE L'INVENTION

### Domaine de l'invention

La présente invention concerne un ensemble pour la pose/dépose d'une pompe cardiaque dans un manchon fixé dans une ouverture d'une paroi ventriculaire.

Elle concerne également une pompe cardiaque pour l'assistance ventriculaire d'un cœur battant, cette pompe cardiaque ayant un corps de pompe configuré pour coopérer avec cet ensemble pour la pose/dépose d'une pompe cardiaque afin de permettre la préhension et le déplacement de celle-ci.

### Arrière-plan technologique

L'insuffisance cardiaque (IC), est un état pathologique dans lequel le cœur d'un patient présente une incapacité à fournir un débit sanguin nécessaire aux besoins métaboliques de l'organisme.

Il est connu pour traiter l'insuffisance cardiaque d'implanter un dispositif d'assistance ventriculaire (DAV), qui est une pompe cardiaque artificielle.

Cette pompe mécanique ne remplace pas le cœur qui continue à fonctionner, mais apporte une aide au ventricule affaibli afin d'accroître le débit sanguin de façon adaptée aux besoins de l'individu.

Cette assistance peut être temporaire dans l'attente d'un greffon disponible pour réaliser une transplantation cardiaque.

Cependant, on observe une proportion significative de patients qui ne recevront pas un tel greffon, soit parce qu'ils ne peuvent être candidats à une telle transplantation, par exemple en raison d'une insuffisance cardiaque sévère, soit parce qu'aucun greffon adapté n'est disponible pour ces patients.

Dans ce cas, l'assistance ventriculaire est utilisée en destination, c'est-à-dire que la pompe cardiaque artificielle est implantée à long terme.

Ces pompes cardiaques font donc l'objet d'intenses recherches visant à améliorer la survie et la qualité de vie des patients présentant une insuffisance cardiaque.

De nombreuses avancées ont été réalisées ces dernières années et on connaît aujourd'hui des dispositifs d'assistance ventriculaire plus compacts, silencieux et présentant une durée de service accrue.

Les pompes cardiaques implantables de l'état de l'art sont ainsi typiquement équipées d'un moteur électrique intégré pour assurer leur fonctionnement, la vitesse de rotation de la pompe fournissant la force nécessaire pour faire circuler le sang depuis le ventricule affaibli vers la circulation corporelle.

On connaît des systèmes d'implantation de telles pompes dans un orifice d'une paroi ventriculaire.

Ces systèmes d'implantation comprennent généralement une portion tubulaire aux extrémités de laquelle sont placés, ou formés, des collerettes destinées à être plaquées chacune contre une face opposée de la paroi ventriculaire après introduction de la portion tubulaire dans un orifice réalisé dans cette paroi ventriculaire.

Ces collerettes permettent ainsi de maintenir en position cette portion tubulaire creuse, laquelle définit alors un conduit ouvert traversant la paroi ventriculaire.

A l'extrémité de cette portion tubulaire qui est placée à l'extérieur du cœur, est montée une pompe aspirative qui assure le renvoi du sang présent dans le ventricule vers la circulation corporelle.

US 2015/258260 A1 divulgue un système et des dispositifs d'insertion percutané de micro-pompes.
US 2016/175501 A1 divulgue un procédé de positionnement de pompe cardiaque par chirurgie invasive avec l'assistance d'un fil-guide.

Bien que représentant un progrès certain pour la qualité de vie d'un patient souffrant d'insuffisance cardiaque, de nombreux inconvénients sont encore constatés.

Notamment, on observe avec ces systèmes d'implantation de l'art antérieur des dommages qui sont causés à la paroi ventriculaire lors de leur installation, lesquels peuvent entraîner des déchirures localisées de cette paroi.

A titre purement illustratif, on connaît un tel système d'implantation dont une des brides vient être plaquée contre une face de la paroi ventriculaire par son déplacement le long de la surface externe de la portion tubulaire. On constate alors que l'opérateur ne pouvant contrôler de manière précise les efforts appliqués sur la paroi ventriculaire lors de la mise en aboutement de cette bride contre la face de la paroi, cette dernière vient typiquement écraser la paroi et abîmer celle-ci.

Par ailleurs, les dimensions des collerettes étant réduites pour autoriser leur passage au travers de l'orifice, la tenue mécanique du système d'implantation est limitée et n'autorise pas, par exemple, l'application d'efforts importants sur celui-ci une fois en place sur la paroi ventriculaire.

Egalement, la pompe étant placée à l'extrémité de la portion tubulaire, extérieure au cœur, et le sang présent dans le ventricule passant dans le conduit ouvert, il existe un risque de perte de sang, notamment en raison des efforts appliqués sur la paroi ventriculaire.

Au surplus, le positionnement de la pompe par rapport à la valve aortique étant imparfait, le rendement hémodynamique n'est pas optimisé et l'expulsion du sang n'est pas dirigée.

En outre, l'installation et le retrait et/ou le remplacement d'une telle pompe cardiaque sont complexes, avec notamment des risques accrues de perte de sang.

Il existe donc un besoin pressant non seulement pour un dispositif de fixation sur un orifice d'une paroi ventriculaire dont le design original surmonte les inconvénients décrits ci-dessus, mais également pour un ensemble pour l'installation/le retrait ou le remplacement d'une pompe cardiaque sur un tel dispositif de fixation.

### Objet de l'invention

La présente invention vise à pallier les inconvénients de l'art antérieur et à répondre aux contraintes ci-dessus énoncées en proposant notamment un ensemble pour la pose/dépose d'une pompe cardiaque dans un dispositif de conduit conçu pour être placé dans une paroi ventriculaire au travers d'une ouverture réalisée dans cette paroi, cet ensemble de pose/dépose étant particulièrement simple dans sa conception et dans son mode opératoire, fiable et autorisant une préhension ferme et sécurisée de cette pompe cardiaque en vue de sa manipulation.

Un autre objet de la présente invention est un tel un ensemble pour la pose/dépose d'une pompe cardiaque autorisant des opérations de maintenance facilitées sur cette pompe cardiaque, ou un remplacement aisée de cette dernière, alors que le cœur du patient continue de battre.

La présente invention vise également une pompe cardiaque spécialement conçue pour être mise en œuvre avec cet ensemble pour la pose/dépose d'une pompe cardiaque.

### BREVE DESCRIPTION DE L'INVENTION

A cet effet, la présente invention concerne un ensemble pour la pose/dépose d'une pompe cardiaque dans un manchon fixé dans une ouverture d'une paroi ventriculaire, ledit ensemble comprenant un élément de guidage présentant une extrémité distale, une extrémité proximale et une lumière s'étendant entre et débouchant auxdites extrémités distale et proximale, ladite pompe cardiaque ayant un corps de pompe.

Selon l'invention, ledit corps de pompe comprenant un élément d'assemblage, ledit ensemble comprend un organe de préhension apte à coulisser dans ladite lumière, ledit organe de préhension comportant à son extrémité libre, une partie d'assemblage complémentaire dudit élément d'assemblage, laquelle est configurée pour coopérer avec ledit élément d'assemblage et joindre cette extrémité libre au corps de pompe afin de permettre la préhension et le déplacement de ladite pompe cardiaque.
Cet ensemble de pose/dépose d'une pompe cardiaque peut avantageusement être mis en oeuvre avec tout manchon fixé dans une ouverture d'une paroi ventriculaire. Par exemple, il est compatible avec des manchons de l'état de l'art, se présentant sous la forme d'un conduit creux comportant à chacune de leurs extrémités, un élément de liaison à la paroi ventriculaire.
Cet organe de préhension permet ainsi manipuler et de déplacer la pompe cardiaque dans la lumière de l'élément de guidage jusque dans le manchon.

Dans différents modes de réalisation particuliers de cet ensemble de pose/dépose, chacun ayant ses avantages particuliers et susceptibles de nombreuses combinaisons techniques possibles:
- ledit élément d'assemblage étant un creux, ou une saillie, ménagés sur ou dans le corps de pompe, ladite partie d'assemblage complémentaire est une tête de forme conjuguée audit creux, respectivement un creux de forme conjuguée à ladite saillie, pour l'assemblage de ladite extrémité libre audit corps de pompe.
- l'extrémité libre dudit organe de préhension comporte un profil polygonal complémentaire d'un profil en creux placé à une extrémité du corps de pompe cardiaque de sorte que cette extrémité libre puisse s'insérer dans ledit profil en creux.
- ledit corps de pompe comportant au moins deux éléments d'assemblage, ladite extrémité libre dudit organe de préhension comprend pour chaque élément d'assemblage, une partie d'assemblage complémentaire de l'élément d'assemblage correspondant.
Ces éléments d'assemblage qui peuvent être identiques ou non, sont par exemple placés sur des côtés opposés dudit corps de pompe.
- ledit corps de pompe comprenant un orifice débouchant dans au moins un logement de réception/retenue du corps de pompe, l'extrémité libre dudit organe de préhension comporte une tête manœuvrable en rotation, laquelle présente un ou plusieurs reliefs d'indexage définissant, par rotation de la tête, une position déverrouillée du corps de pompe et de l'extrémité de l'organe de préhension pour permettre leur séparation, ladite tête pouvant librement traverser ledit orifice pour pénétrer ou sortir dudit logement, et une position verrouillée, dans laquelle le ou les reliefs d'indexage sont en prise dans le logement de réception/retenue pour assurer un assemblage mécanique de ladite extrémité de l'organe de préhension et de ladite pompe cardiaque.
- ledit organe de préhension est un corps allongé de forme générale cylindrique, de telle sorte qu'il puisse être entraîné en rotation dans la lumière dudit élément de guidage.

A titre purement illustratif, cette mise en rotation de l'organe de préhension peut servir à visser/dévisser ledit corps de pompe sur ledit filetage porté par la paroi intérieure dudit manchon.
- cet ensemble de pose/dépose comporte un élément d'étanchéité placé à l'intérieur de ladite lumière, et de préférence dans sa partie distale, cet élément d'étanchéité étant ouvert lorsque l'extrémité libre dudit organe de préhension est pressée contre celui-ci, afin de libérer le passage audit organe de préhension au travers de cet élément d'étanchéité.
- l'extrémité distale dudit élément de guidage comporte un dispositif de stabilisation d'une partie du cœur, ledit dispositif de stabilisation comportant une partie évasée comprenant, de préférence à son extrémité de plus large diamètre, une rainure, laquelle présente au moins un orifice d'aspiration pour créer un vide dans ladite rainure lorsqu'une portion de paroi ventriculaire est en contact avec ledit dispositif de stabilisation.
- cet ensemble de pose/dépose comporte un ou plusieurs autres éléments choisis dans le groupe comprenant un outil de perforation, un outil dilatateur, un câble de guidage, ... et des combinaisons de ces éléments, chacun desdits éléments ou leurs combinaisons étant aptes à coulisser dans ladite lumière.

La présente invention concerne également une pompe cardiaque pour l'assistance ventriculaire d'un cœur battant, ladite pompe cardiaque ayant un corps de pompe.

Selon l'invention, le corps de pompe comprend au moins un élément d'assemblage configuré pour coopérer avec l'extrémité libre de l'organe de préhension dudit ensemble pour la pose/dépose d'une pompe cardiaque tel que décrit précédemment, afin de permettre la préhension et le déplacement de ladite pompe cardiaque.
Cette pompe est donc spécialement conçue pour être mise en œuvre avec le dispositif de pose/dépose décrit plus haut. Elle présente donc un lien avec cet ensemble de pose/dépose et est destinée à fonctionner avec dernier.

Dans différents modes de réalisation particuliers de cette pompe cardiaque, chacun ayant ses avantages particuliers et susceptibles de nombreuses combinaisons techniques possibles:
- cette pompe est une pompe cardiaque propulsive.
- ledit manchon s'étendant dans une direction axiale et comprenant une extrémité axiale avant et une extrémité axiale arrière, ledit manchon comprenant un canal intérieur débouchant aux extrémités axiales avant et arrière, au moins une partie de la paroi intérieure dudit manchon délimitant ledit canal intérieur présentant un filetage, ledit corps de pompe comprenant un filetage sur une partie de sa surface externe, lequel est distinct dudit au moins un élément d'assemblage, ledit filetage étant complémentaire du filetage de ladite au moins une partie de la paroi intérieure du manchon pour assurer leur engagement,.
De préférence, ces filetages sont configurés pour assurer l'étanchéité de l'assemblage corps de pompe/manchon.
Alternativement, ledit manchon s'étendant dans une direction axiale et comprenant une extrémité axiale avant et une extrémité axiale arrière, ledit manchon comprenant un canal intérieur débouchant aux extrémités axiales avant et arrière, la paroi intérieure dudit manchon délimitant ledit canal intérieur comporte au moins deux moyens d'encliquetage, ledit corps de pompe comprenant pour chaque moyen d'encliquetage du manchon, un moyen d'encliquetage complémentaire configuré pour coopérer avec un moyen d'encliquetage correspondant du manchon pour assurer l'assemblage du corps de pompe sur la paroi intérieure du manchon, chaque moyen d'encliquetage complémentaire étant placé sur la surface externe du corps de pompe en étant distinct dudit au moins un élément d'assemblage.
De préférence, ledit corps de pompe présente un joint périphérique placé en amont des moyens d'encliquetage pour assurer l'étanchéité de l'assemblage corps de pompe/manchon.
L'amont est repéré par rapport au sens d'écoulement qu'aurait le sang sortant du cœur au travers du manchon.

La présente invention concerne encore un dispositif de fixation sur une ouverture d'une paroi ventriculaire, comprenant
- un corps principal creux de forme générale cylindrique présentant une surface externe,
- une bague reçue à une première extrémité dudit corps creux, cette bague étant mobile le long d'au moins une partie de la surface externe du corps creux,
- une membrane tubulaire recouvrant la surface externe du corps creux en s'étendant entre ladite bague et l'extrémité opposée à la première extrémité du corps creux, dite seconde extrémité,
- une extrémité distale de cette membrane tubulaire, placée du côté de ladite seconde extrémité du corps creux, étant auto-expansible entre une première configuration stable dans laquelle elle présente une forme tubulaire, ou essentiellement tubulaire, et une seconde configuration stable dans laquelle elle définit une première bride s'étendant radialement, ou sensiblement radialement, à partir dudit corps creux, cette première bride étant destinée à venir en aboutement contre une face de ladite paroi ventriculaire,
- l'autre extrémité, dite proximale, de cette membrane tubulaire étant susceptible d'être déformée par le déplacement de ladite bague le long de la surface externe du corps creux de sorte à former une bride de retenue dont la position peut varier par rapport à ladite première bride de manière à s'adapter à des parois ventriculaires d'épaisseurs différentes,
- ladite bride de retenue étant destinée à venir en contact avec la face opposée de ladite paroi ventriculaire de sorte que lesdites brides et l'ensemble formé par la partie du corps creux et la portion de la membrane tubulaire la recouvrant, traversant ladite paroi ventriculaire au travers de ladite ouverture, bloquent en position ledit dispositif de fixation dans ladite ouverture.

Cette bague, dite de pressage, présente une forme adaptée pour s'engager sur la surface externe dudit corps creux. Elle est placée à, ou proche de, ladite première extrémité du corps creux.

De manière avantageuse, cette bague permet de mettre en compression la membrane tubulaire placée sur la surface externe dudit corps creux. Le degré de mise en compression, ou pressage, de la membrane tubulaire est contrôlé par l'opérateur en déplaçant plus ou moins la bague le long de la surface externe du corps creux. On contrôle ainsi avantageusement les efforts appliqués sur la paroi ventriculaire.

Elle présente à cet effet sur son bord destiné à venir en contact avec l'extrémité proximale de ladite membrane tubulaire, une surface annulaire de pressage de ladite membrane tubulaire. Cette surface annulaire peut comporter un logement pour recevoir l'extrémité proximale de la membrane tubulaire.

L'extrémité proximale de la membrane tubulaire est donc susceptible d'être déformée en s'évasant vers l'extérieur de manière à former une bride de retenue écartée de la première bride. Cette bride élargie radialement présente avantageusement une surface planaire ou sensiblement planaire destinée à faire face à la paroi ventriculaire.

Le corps creux présente une dimension longitudinale supérieure à celle de l'ouverture s'étendant entre les faces opposées de la paroi ventriculaire et un diamètre égal ou supérieur à celui de l'ouverture dans la paroi ventriculaire.

Ainsi, et de manière avantageuse, le dispositif de fixation sur une ouverture d'une paroi ventriculaire assure une étanchéité après pose sans réalisation d'aucune suture, contrairement aux dispositifs de l'état de l'art. En ce sens, il s'agit d'un dispositif de fixation d'une ponction ventriculaire d'un diamètre supérieure à 20 mm, ne nécessitant aucun point de suture.

Dans différents modes de réalisation particuliers de ce dispositif, chacun ayant ses avantages particuliers et susceptibles de nombreuses combinaisons techniques possibles:
- l'extrémité proximale de la membrane tubulaire est configurée pour être progressivement déformée lors du déplacement de la bague de manière à former une bride de retenue dont la position peut varier dans une plage prédéterminée de distances à partir de la première bride.
- ladite bague comporte sur son pourtour au moins un doigt d'accouplement destiné à coopérer avec des crans placés sur la surface externe dudit corps creux. On obtient ainsi un déplacement discret, ou ponctuel, de la bague le long de la surface externe du corps creux.
Alternativement, la surface externe dudit corps creux comprenant un filetage sur au moins une partie de sa surface externe, la surface interne de ladite bague comporte un filetage configuré pour coopérer avec ledit filetage placé sur la surface externe du corps creux afin de permettre le déplacement de ladite bague.
- ladite bague comporte un doigt anti-retour pour empêcher un éventuel desserrement de la bague après formation de la bride de retenue.
- la surface externe du corps creux comporte une butée pour stopper le déplacement de ladite bague,
- ladite bague et ledit corps creux sont réalisés dans des matériaux rigides et inertes pour le corps humain,
- ladite membrane tubulaire est réalisée en nitinol, dans un alliage de nitinol ou encore en polyuréthane expansible,
- le dispositif est configuré pour former une bride de retenue ayant un diamètre externe strictement supérieur 3/2 D où D est le diamètre de l'ouverture dans la paroi ventriculaire.
De préférence, la bride de retenue ainsi formée présente un diamètre externe compris entre 3/2 D et 3 D.
A titre purement illustratif, cette bride de retenue présente un diamètre de 40 mm.
Cette grande dimension de la bride de retenue destinée à être ainsi formée, renforce l'appui du dispositif sur la paroi ventriculaire et assure, de ce fait, une meilleure tenue en position, ou stabilisation, du dispositif de fixation sur cette paroi ventriculaire notamment lors d'une intervention sur la pompe cardiaque propulsive destinée à être reçue dans le canal de ce corps principal.

Par ailleurs, d'éventuelles contraintes sont réparties sur une plus grande surface de la paroi ventriculaire de sorte que le risque d'endommager cette paroi est réduit, voire supprimé.
- la paroi interne du corps creux délimitant un canal s'étendant entre les première et seconde extrémités du corps creux, ladite paroi interne comporte des moyens pour l'assemblage étanche du corps d'une pompe cardiaque et de ladite paroi, ledit corps de pompe étant ainsi reçu au moins en partie dans ledit canal.
Le diamètre du canal est égal ou sensiblement égal au diamètre externe du corps de pompe pour loger cette dernière dans ledit canal.
De préférence, le corps de la pompe destinée à être logée dans le canal délimité par la paroi interne du corps creux comportant un filetage sur sa surface externe, cette paroi interne comprend un filetage destiné à coopérer avec le filetage du corps de pompe.
- ladite pompe cardiaque est un dispositif d'assistance ventriculaire (DAV) implantable.

De préférence, il s'agit d'une pompe cardiaque propulsive.

Cette pompe cardiaque étant ancrée à la paroi du cœur au moyen du dispositif de fixation, le patient peut dès lors se déplacer de manière active sans aucun risque.

La première extrémité du corps principal creux comporte une ouverture en communication avec le canal délimité par la paroi intérieure du corps creux. De manière avantageuse, une ou plusieurs liaisons filaires telles que des câbles, peuvent passer au travers de cette ouverture pour relier une pompe cardiaque artificielle logée dans ce canal à une unité de gestion de cette pompe. Des signaux de commande de cette pompe peuvent ainsi être envoyés à la pompe artificielle.

Cette unité de gestion peut également comprendre un émetteur-récepteur sans fil pour transmettre automatiquement des données telles que des informations sur le rythme cardiaque ou encore l'état de la source d'alimentation implantée, en vue d'un suivi de télémédecine.

La transmission des données peut être réalisée vers un terminal externe portatif au moyen de signaux de communication sans fil à courte portée, par exemple basés sur un protocole bluetooth ou Zigbee, .... Ce terminal externe peut comporter un moyen de communication mettant en œuvre un réseau d'accès cellulaire et/ou un réseau internet pour transmettre ces données vers par exemple un cardiologue. Le réseau d'accès cellulaire peut être de plusieurs types (2G, 3G, 4G), chaque type de réseau étant accessible selon plusieurs technologies d'accès cellulaires (2G : EDGE, GPRS, 3G : UMTS, HSDPA, HSUPA, HSPA, HSPA+, 4G : LTE). Le réseau internet est par exemple un réseau comportant des points d'accès non cellulaires sans fil tel qu'un réseau WLAN, par exemple Wi-Fi ou WiMAX ou encore d'un réseau Li-Fi. Ce terminal externe peut présenter un dispositif d'affichage pour permettre à l'utilisateur de lire des messages ou de choisir des options dans un menu.

De préférence, cette unité centrale comprend une ou plusieurs entrées pour recevoir un ou plusieurs signaux dont chacun est lié à une vibration mécanique audible ou inaudible liée à l'activité mécanique du cœur, ladite unité centrale comprenant un premier sous-ensemble d'instructions logicielles dudit ensemble d'instructions logicielles qui lorsqu'elles sont exécutées par ledit processeur, sont configurées pour définir une fenêtre temporelle de mesure dudit ou desdits signaux, pour analyser chaque signal ainsi reçu à l'entrée de ladite unité centrale durant cette fenêtre temporelle afin de déterminer un ou plusieurs paramètres du signal correspondant, pour comparer le ou les paramètres de chaque signal ainsi déterminés avec une ou plusieurs données préalablement enregistrées dans une unité de stockage de ladite unité centrale afin d'identifier le signal correspondant à la fermeture de la vanne mitrale ainsi que l'instant t₁ correspondant à la fermeture de ladite valve mitrale.

La présente invention concerne également une unité d'assistance ventriculaire comprenant une pompe cardiaque propulsive et un dispositif de fixation sur une ouverture dans une paroi ventriculaire, tel que décrit précédemment.

De manière avantageuse, cette pompe cardiaque propulsive est logée à l'intérieur du canal délimité par la paroi interne du corps principal creux. De préférence, le corps de pompe et la paroi interne du corps principal creux sont assemblés de manière étanche pour éviter tout reflux de sang.

### BREVE DESCRIPTION DES DESSINS

D'autres avantages, buts et caractéristiques particulières de la présente invention ressortiront de la description qui va suivre, faite, dans un but explicatif et nullement limitatif, en regard des dessins annexés, dans lesquels :
- la Figure 1 représente schématiquement un dispositif d'ancrage sans suture pour une pompe cardiaque propulsive selon un mode de réalisation particulier de l'invention, l'extrémité distale de la membrane tubulaire étant dans sa première configuration stable, et la bague n'ayant pas encore été déplacée le long de la surface externe du tube pour mettre en compression l'extrémité proximale de la membrane tubulaire ;
- la Figure 2 montre le dispositif d'ancrage de la Fig. 1 dans un état activé, dans lequel l'extrémité distale de la membrane tubulaire est dans sa deuxième configuration stable et l'extrémité proximale de la membrane tubulaire a été déformée pour définir une bride de retenue, les faces opposées d'une paroi ventriculaire (PV) ont été schématisées dans la partie supérieure du dispositif pour illustrer la mise en contact des brides sur ces faces opposées, l'espace extra-cardiaque (EC) étant également rappelé ;

- la Figure 3 est une représentation schématique partielle et en coupe du dispositif d'ancrage illustré à la Fig. 1 ;
- la Figure 4 est une représentation schématique partielle et en coupe du dispositif d'ancrage illustré à la Fig. 2 ;
- la Figure 5 est une vue en perspective de la bague du dispositif d'ancrage de la Fig. 1 montrant le clapet anti-retour de la bague ;
- la Figure 6 est une vue en coupe longitudinale d'un ensemble de pose/dépose d'une pompe cardiaque reliée à l'apex d'un cœur battant, selon un mode de réalisation particulier de l'invention, cet ensemble étant montré dans une première configuration dans laquelle il comprend un outil de perforation et d'ablation d'une partie de la paroi ventriculaire pour réaliser une ouverture dans ladite paroi en vue de la pose d'un manchon dans cette ouverture ;
- la Figure 7 est une vue élargie et en coupe longitudinale de l'ensemble de pose/dépose de la Fig. 6, l'extrémité de l'outil de perforation et d'ablation comportant un capuchon de protection de son aiguille avant passage de la vanne clapet ;
- la Figure 8 est une vue en coupe longitudinale et partielle de l'ensemble de pose/dépose de la Fig. 6, selon une autre configuration, dans laquelle cet ensemble comporte un organe de préhension, l'extrémité duquel est reliée à une pompe cardiaque engagée dans un dispositif d'ancrage placé dans l'ouverture d'une paroi ventriculaire et traversant celle-ci, l'extrémité distale de la membrane tubulaire étant dans sa deuxième configuration ;
- la Figure 9 est une autre représentation de l'ensemble de pose/dépose dans sa configuration illustrée à la Fig. 8, montrant également le déploiement de l'extrémité distale de la membrane tubulaire à l'intérieur de l'apex ;
- la Figure 10 est une vue en coupe longitudinale de l'ensemble de pose/dépose d'une pompe cardiaque de la Fig. 8 dans lequel la bague de mise en compression de la membrane tubulaire a été déplacée le long de la surface externe du tube pour former une bride de retenue, le dispositif d'ancrage étant ainsi fixé dans l'ouverture de la paroi ventriculaire, une partie de la pompe cardiaque étant intraventriculaire ;
- la Figure 11 est une autre représentation de l'ensemble de pose/dépose dans sa configuration illustrée à la Fig. 10, montrant également le déploiement de l'extrémité proximale de la membrane tubulaire sur la surface externe de l'apex ;
- la Figure 12 montre le dispositif d'ancrage fixé dans l'ouverture de la paroi ventriculaire, la pompe étant vissée dans le canal intérieur de ce dispositif d'ancrage, l'ensemble de pose/dépose ayant été retiré ;
- la Figure 13 montre le dispositif d'ancrage fixé dans l'ouverture de la paroi ventriculaire, la pompe étant vissée dans le canal intérieur de ce dispositif d'ancrage, un bouchon étant placé à l'extrémité du dispositif d'ancrage placée hors du cœur pour obturer celle-ci, ce bouchon comportant un orifice pour le passage de l'alimentation de la pompe cardiaque ;

### DESCRIPTION DETAILLEE DE MODE DE REALISATION DE L'INVENTION

Tout d'abord, on note que les figures ne sont pas à l'échelle.

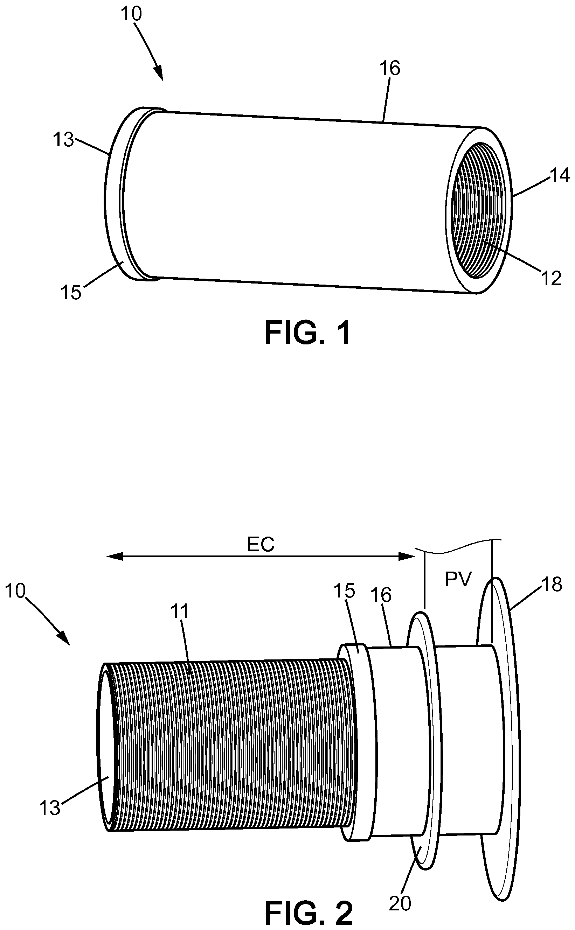

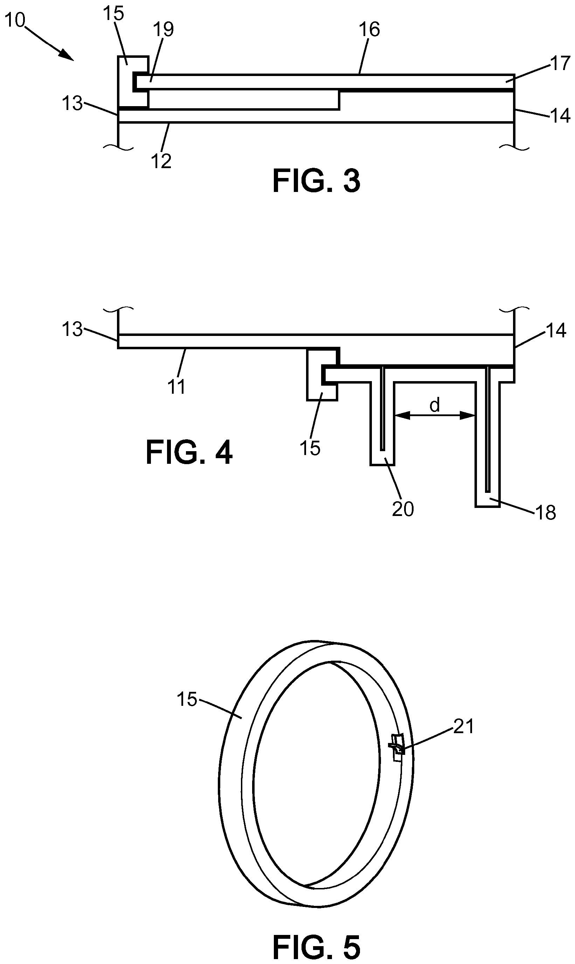

Les Figures 1 à 5 montrent de manière schématique un dispositif d'ancrage 10 sans suture d'une pompe cardiaque propulsive dans une ouverture d'une paroi ventriculaire, selon un mode de réalisation particulier de la présente invention.

Ce dispositif d'ancrage 10 comporte un tube présentant une surface externe 11 et une surface intérieure 12 délimitant un canal s'étendant entre une première extrémité 13 et une seconde extrémité 14, ouvertes de ce tube.

Les surfaces externe 11 et intérieure 12 de ce tube 10 présentent un filetage, la surface externe 11 du tube comprenant du côté de sa seconde extrémité 14, une portion de paroi présentant une surépaisseur, laquelle est non filetée et reliée par un épaulement au reste de la surface externe 11 du tube filetée. Cet épaulement définit ainsi une butée pour limiter le déplacement d'une bague 15 de pressage reçue à la première extrémité 13 du tube.

La surface interne de cette bague 15 comporte un filetage (non représenté) destiné à coopérer avec le filetage de la surface externe 11 du tube pour autoriser un déplacement continu de cette bague 15 le long d'une partie de la surface externe 11 de ce tube.

Entre cette bague 15 et la seconde extrémité 14 du tube est placée une membrane tubulaire 16 recouvrant la surface externe 11 du tube.

La bague 15 comporte sur sa face destinée à presser la membrane tubulaire 16, un logement tel qu'une rainure, pour recevoir l'extrémité libre correspondante de la membrane tubulaire 16. De manière avantageuse, cette extrémité de la membrane tubulaire 16, encore appelée extrémité proximale, est non solidarisée dans son logement pour éviter les torsions de la membrane.

Une extrémité 17 distale de cette membrane tubulaire 16, placée du côté de la seconde extrémité 14 du tube, est auto-expansible entre une première configuration stable dans laquelle elle présente une forme tubulaire, et une seconde configuration stable dans laquelle elle définit une première bride 18 s'étendant radialement à partir dudit tube.

La première configuration stable de la membrane permet avantageusement une introduction aisée de la seconde extrémité 14 du tube au travers de l'ouverture de la paroi ventriculaire.

La seconde configuration stable de la membrane tubulaire 16 permet de générer une première bride 18 de sorte que celle-ci vienne en aboutement, ou encore soit plaquée, contre une face de la paroi ventriculaire lorsque le tube a été introduit au travers de l'ouverture de cette paroi ventriculaire.

Le passage de la première configuration stable à la seconde configuration stable est obtenu par une augmentation de la température de la membrane tubulaire 16, par exemple, en exposant celle-ci à la température du corps humain, la membrane tubulaire 16 étant réalisée en nitinol ou en polyuréthane expansé - matériel à mémoire de forme.

L'extrémité opposée, dite proximale 19, de cette membrane tubulaire 16 est susceptible d'être déformée progressivement par le déplacement de la bague 15 le long de la surface externe 11 du tube de manière à former une seconde bride 20 de retenue dont la position peut varier dans une plage prédéterminée de distances d à partir de la première bride 18 définie par l'extrémité distale 17 de la membrane tubulaire 16 dans sa seconde configuration stable. Cette distance d permet de comprimer la paroi ventriculaire afin d'assurer l'étanchéité du dispositif et un maintien fiable et durable.

Il est ainsi possible d'adapter le dispositif d'ancrage 10 à des parois ventriculaires d'épaisseurs différentes. L'épaulement déterminé par le profil de la surface 11 externe du tube permet avantageusement de définir une limite supérieure sur la mise en compression de la membrane tubulaire 16 et ainsi de limiter les efforts appliqués sur la paroi ventriculaire.

De manière avantageuse, cette bague 15 comporte un doigt 21 anti-retour.

Ce doigt 21 anti-retour permet d'empêcher un éventuel desserrement de la bague 15 après formation de la seconde bride 20 de retenue, lequel serait susceptible d'entraîner un relâchement de, ou une libération partielle des contraintes dans, la membrane tubulaire 16, ayant pour conséquence, un affaissement de la seconde bride 20 de retenue. Une telle déformation de la seconde bride 20 de retenue serait susceptible d'entraîner une moins bonne étanchéité de la liaison entre le dispositif d'ancrage et la paroi ventriculaire susceptible de résulter en des fuites de sang.

La bague 15 et le tube sont réalisés dans des matériaux rigides et inertes, c'est-à-dire biocompatibles avec l'organisme humain. Ils sont, par exemple, réalisés en PEEK (polyétheréthercétone), en céramique ou en titane. Ces éléments peuvent être imprimés, c'est-à-dire qu'ils sont alors formés par un procédé d'impression tridimensionnelle.

Une fois, ce dispositif d'ancrage 10 solidarisé à la paroi ventriculaire, il est possible d'introduire dans le canal délimité par la surface intérieure 12 du tube, une pompe cardiaque propulsive.

Cette pompe (non représentée) est avantageusement destinée à être reçue dans le canal du tube de sorte qu'elle ne forme pas saillie du tube à l'extérieur du cœur.

En outre, l'assemblage du corps de pompe et de la surface 12 intérieure du tube est étanche pour empêcher tout reflux sanguin par ce canal. Un tel assemblage est avantageusement réalisé ici par vissage du corps de pompe sur le filetage porté par la surface intérieure 12 du tube.

Pour cela, le corps de pompe présente sur au moins une partie de sa surface externe 11 un filetage destiné à coopérer avec le filetage porté par la surface 12 intérieure du tube.

Le vissage de la pompe au sein de la surface intérieure 12 du tube permet également de régler, ou ajuster, le positionnement de cette pompe vis-à-vis de la valve aortique et, par conséquent, d'optimiser la position de celle-ci pour atteindre un meilleur rendement hémodynamique. De manière avantageuse, on obtient ainsi un débit cardiaque optimisé, lequel est permis par la possibilité de réaliser un déplacement continu, et par conséquent, extrêmement précis, du corps de pompe le long du filetage formé à la surface intérieure 12 du tube.

Ainsi, et de manière plus générale, la présente invention concerne également un procédé visant à optimiser le rendement hémodynamique, dans lequel on ajuste la position d'une pompe cardiaque implantée sur une paroi ventriculaire par rapport à la valve aortique du patient. De préférence, cet ajustement est obtenu par un déplacement de la pompe cardiaque par rapport à cette valve aortique. Et encore mieux, cet ajustement est réalisé par vissage/dévissage du corps de la pompe le long d'une surface filetée interne, ou encore sur le côté intérieur fileté, d'une paroi tubulaire d'un dispositif de fixation, ou d'ancrage, fixé dans une ouverture d'une paroi ventriculaire, le corps de pompe comprenant, à cet effet, sur sa surface externe un filetage complémentaire du filetage du côté intérieur fileté de la paroi tubulaire et destiné à coopérer avec ce dernier. Un tel ajustement en position est avantageusement très précis en raison du déplacement continu de la pompe cardiaque, autorisé par ces filetages.

On cherche ainsi à positionner l'extrémité de la pompe cardiaque pour diriger le sang propulsé par cette pompe vers la valve aortique du patient. De préférence, on cherche à placer cette extrémité de la pompe cardiaque à une distance comprise entre 10 mm et 20 mm de la valve aortique.

Afin de limiter le déplacement de la pompe cardiaque par vissage de cette dernière le long de la surface intérieure 12 du tube, cette surface intérieure 12 du tube peut comporter au moins une butée. On peut ainsi contrôler l'avancée intraventriculaire de la pompe cardiaque.

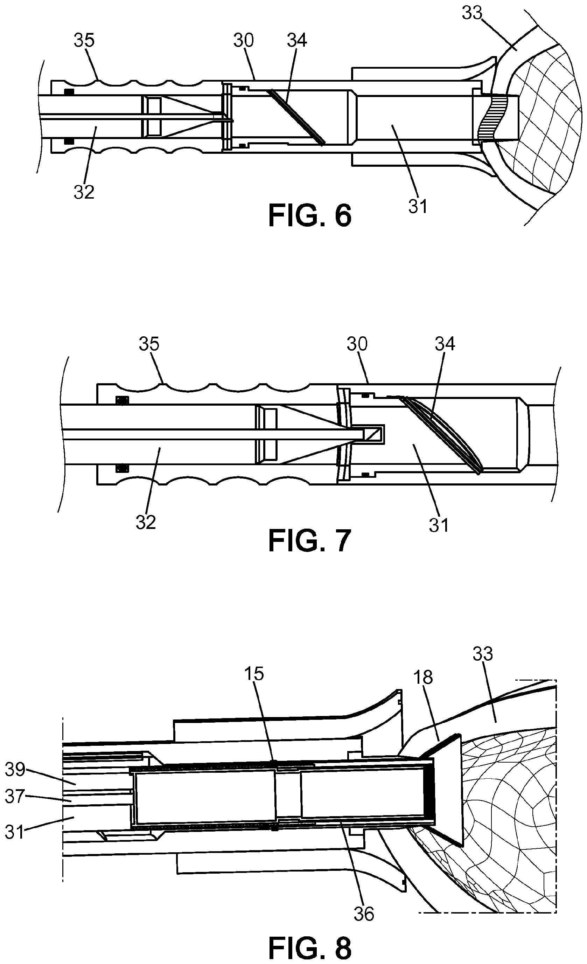

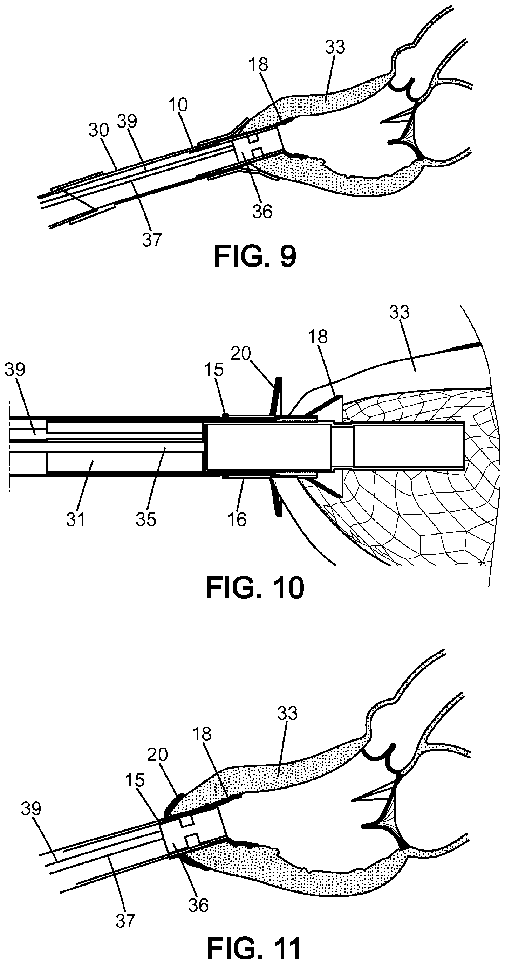

Les Figures 6 à 13 illustrent un système pour l'introduction d'un dispositif médical tel qu'une aiguille, un dispositif d'ancrage 10 sur un orifice d'une paroi ventriculaire tel que décrit ci-dessus, une pompe cardiaque ou encore un organe de préhension de cette pompe, selon un mode de réalisation particulier de la présente invention.

Ce système pour l'introduction d'un dispositif médical comprend un corps 30 principal définissant un canal 31 intérieur longitudinal, ou lumière, pour recevoir ce dispositif médical 32, ce dispositif médical étant mobile en translation dans ce canal 31 intérieur de sorte qu'une partie de celui-ci peut être placée en saillie de ce corps 30 principal pour son introduction dans une paroi ventriculaire 33 ou dans le canal délimité par la paroi intérieure 12 d'un manchon fixé dans l'ouverture de la paroi ventriculaire 33, tel que celui d'un dispositif d'ancrage décrit plus haut.

Ce corps 30 principal comprend également un élément d'étanchéité 34 placé à l'intérieur de ce canal de telle sorte que cet élément d'étanchéité 34 peut être ouvert lorsque l'extrémité du dispositif médical est pressée contre celui-ci, afin de libérer le passage au dispositif médical 32 à travers cet élément d'étanchéité 34.

Cet élément d'étanchéité 34 comprend ici une vanne clapet dont le siège est incliné à 45° pour faciliter le passage du dispositif médical 32, tout en empêchant le passage du sang dans l'autre direction lorsque cette vanne clapet est dans sa position d'obturation. Cette vanne clapet forme ainsi une vanne anti-retour.

L'extrémité proximale du corps 30 principal comporte une poignée 35 de préhension et l'extrémité distale du corps principal comporte avantageusement une valve de purge (non représentée) afin d'éliminer toute présence d'air dans la partie du canal intérieur placée en aval de l'élément d'étanchéité 34.

Afin de manipuler la pompe cardiaque 36 pour son implantation dans, ou son retrait, du canal délimité par la paroi intérieure 12 du tube, ce système comporte un organe 37 de préhension apte à coulisser dans le canal 31 intérieur, cet organe 37 de préhension présentant à son extrémité libre, une partie complémentaire d'une empreinte portée par le corps de pompe.

Il est ainsi possible d'assurer l'engagement de l'extrémité libre de cet organe 37 de préhension et de l'empreinte du corps de pompe pour la préhension et la manipulation de la pompe cardiaque 36.

A titre purement illustratif, le corps de pompe présentant une empreinte polygonale creuse telle qu'à six pans ménagés dans un creux du corps de pompe, l'extrémité libre de l'organe 37 de préhension présente une forme complémentaire telle qu'une forme mâle hexagonale.

Cet organe 37 de préhension se présente avantageusement sous la forme d'une tige que l'opérateur peut manipuler par son extrémité proximale de manière à entraîner en rotation celle-ci afin de visser, ou dévisser, le corps de pompe dans son logement défini par le canal délimité par la paroi intérieure 12 du tube.

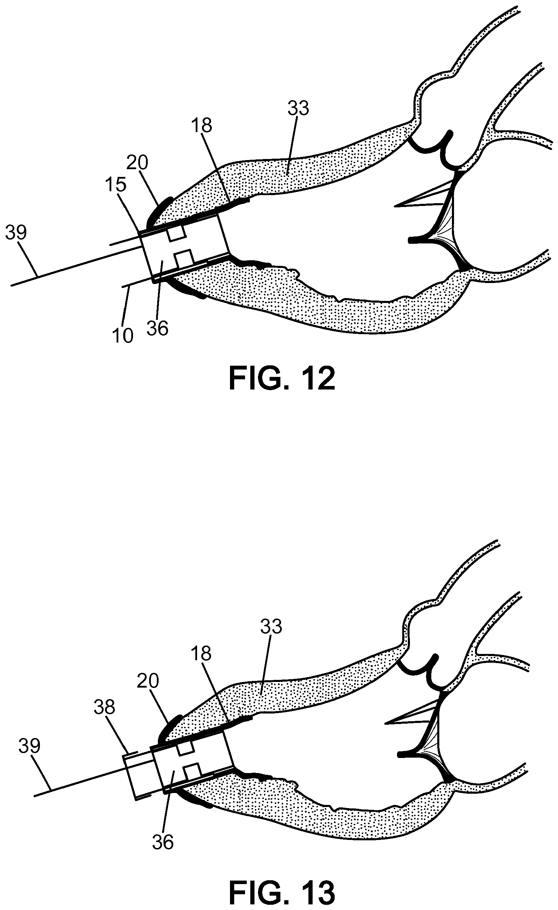

La Figure 13 montre le dispositif d'ancrage fixé dans l'ouverture de la paroi ventriculaire, la pompe cardiaque 36 étant vissée dans le canal intérieur de ce dispositif d'ancrage, un bouchon 38 étant placé à l'extrémité du dispositif d'ancrage placée hors du cœur pour obturer celle-ci, ce bouchon 38 comportant un orifice pour le passage de l'alimentation 39 de la pompe cardiaque 36.

## Revendications

1. Ensemble pour la pose/dépose d'une pompe cardiaque (36) dans un manchon fixé dans une ouverture d'une paroi ventriculaire (33), ledit ensemble comprenant un élément de guidage (30) présentant une extrémité distale, une extrémité proximale et une lumière (31) s'étendant entre et débouchant auxdites extrémités distale et proximale, ladite pompe cardiaque (36) ayant un corps de pompe,
**caractérisé en ce que** ledit corps de pompe comprenant un élément d'assemblage, ledit ensemble comprend un organe de préhension (37) apte à coulisser dans ladite lumière (31), ledit organe de préhension (37) comportant à son extrémité libre, une partie d'assemblage complémentaire dudit élément d'assemblage, laquelle est configurée pour coopérer avec ledit élément d'assemblage et joindre cette extrémité libre au corps de pompe afin de permettre la préhension et le déplacement de ladite pompe cardiaque (36) dans ladite lumière (31) dudit élément de guidage (30) dudit ensemble jusqu'audit manchon.

2. Ensemble selon la revendication 1, **caractérisé en ce que** ledit élément d'assemblage étant un creux, ou une saillie, ménagés sur ou dans le corps de pompe, ladite partie d'assemblage complémentaire est une tête de forme conjuguée audit creux, respectivement un creux de forme conjuguée à ladite saillie, pour l'assemblage de ladite extrémité libre audit corps de pompe.

3. Ensemble selon la revendication 1 ou 2, **caractérisé en ce que** l'extrémité libre dudit organe de préhension (37) comporte un profil polygonal complémentaire d'un profil en creux placé à une extrémité du corps de pompe cardiaque (36) de sorte que cette extrémité libre puisse s'insérer dans ledit profil en creux.

4. Ensemble selon l'une quelconque des revendications 1 à 3, **caractérisé en ce que** ledit corps de pompe comportant au moins deux éléments d'assemblage, ladite extrémité libre dudit organe de préhension (37) comprend pour chaque élément d'assemblage, une partie d'assemblage complémentaire de l'élément d'assemblage correspondant.

5. Ensemble selon l'une quelconque des revendications 1 à 3, **caractérisé en ce que** ledit corps de pompe comprenant un orifice débouchant dans au moins un logement de réception/retenue du corps de pompe, l'extrémité libre dudit organe de préhension (37) comporte une tête manœuvrable en rotation, laquelle présente un ou plusieurs reliefs d'indexage définissant, par rotation de la tête, une position déverrouillée du corps de pompe et de l'extrémité de l'organe de préhension (37) pour permettre leur séparation, ladite tête pouvant librement traverser ledit orifice pour pénétrer ou sortir dudit logement, et une position verrouillée, dans laquelle le ou les reliefs d'indexage sont en prise dans le logement de réception/retenue pour assurer un assemblage mécanique de ladite extrémité de l'organe de préhension (37) et de ladite pompe cardiaque (36).

6. Ensemble selon l'une quelconque des revendications 1 à 5, **caractérisé en ce que** ledit organe de préhension (37) est un corps allongé de forme générale cylindrique, de telle sorte qu'il puisse être entraîné en rotation dans la lumière (31) dudit élément de guidage (30).

7. Ensemble selon l'une quelconque des revendications 1 à 6, **caractérisé en ce qu'**il comporte un élément d'étanchéité placé à l'intérieur de ladite lumière (31), et de préférence dans sa partie distale, cet élément d'étanchéité étant ouvert lorsque l'extrémité libre dudit organe de préhension (37) est pressée contre celui-ci, afin de libérer le passage audit organe de préhension (37) au travers de cet élément d'étanchéité.

8. Ensemble selon l'une quelconque des revendications 1 à 7, **caractérisé en ce que** l'extrémité distale dudit élément de guidage (30) comporte un dispositif de stabilisation d'une partie du cœur, ledit dispositif de stabilisation comportant une partie évasée comprenant, de préférence à son extrémité de plus large diamètre, une rainure, laquelle présente au moins un orifice d'aspiration pour créer un vide dans ladite rainure lorsqu'une portion de paroi ventriculaire (33) est en contact avec ledit dispositif de stabilisation.

9. Ensemble selon l'une quelconque des revendications 1 à 8, **caractérisé en ce qu'**il comporte un ou plusieurs autres éléments choisis dans le groupe comprenant un outil de perforation, un outil dilatateur, un câble de guidage, ... et des combinaisons de ces éléments, chacun desdits éléments ou leurs combinaisons étant aptes à coulisser dans ladite lumière (31).

10. Pompe cardiaque pour l'assistance ventriculaire d'un cœur battant, ladite pompe cardiaque (36) ayant un corps de pompe, **caractérisée en ce que** ledit corps de pompe comprend au moins un élément d'assemblage configuré pour coopérer avec l'extrémité libre de l'organe de préhension (37) d'un ensemble pour la pose/dépose d'une pompe cardiaque (36) dans un manchon fixé dans une ouverture d'une paroi ventriculaire selon l'une quelconque des revendications 1 à 9, afin de permettre la préhension et le déplacement de ladite pompe cardiaque (36) dans la lumière (31) de l'élément de guidage (30) dudit ensemble jusqu'audit manchon.

11. Pompe selon la revendication 10, **caractérisée en ce qu'**elle est une pompe cardiaque (36) propulsive.

12. Pompe selon la revendication 10 ou 11, **caractérisée en ce que** ledit manchon (10) comprenant un canal intérieur et ladite pompe étant destinée à être reçue dans la canal dudit manchon, ledit manchon (10) s'étendant dans une direction axiale et comprenant une extrémité axiale avant (14) et une extrémité axiale arrière (13), ledit canal intérieur débouchant aux extrémités axiales avant et arrière, au moins une partie de la paroi intérieure dudit manchon (10) délimitant ledit canal intérieur présentant un filetage, ledit corps de pompe comprend un filetage sur une partie de sa surface externe, lequel est distinct dudit au moins un élément d'assemblage, ledit filetage étant complémentaire du filetage de ladite au moins une partie de la paroi intérieure du manchon (10) pour assurer leur engagement.

13. Pompe selon la revendication 12, **caractérisée en ce que** lesdits filetages sont configurés pour assurer l'étanchéité de l'assemblage corps de pompe/manchon (10).

14. Pompe selon la revendication 10 ou 11, **caractérisée en ce que** ledit manchon (10) comprenant un canal intérieur et ladite pompe étant destinée à être reçue dans la canal dudit manchon, ledit manchon (10) s'étendant dans une direction axiale et comprenant une extrémité axiale avant (14) et une extrémité axiale arrière (13), ledit canal intérieur débouchant aux extrémités axiales avant et arrière, la paroi intérieure dudit manchon (10) délimitant ledit canal intérieur comportant au moins deux moyens d'encliquetage, ledit corps de pompe comprend pour chaque moyen d'encliquetage du manchon (10), un moyen d'encliquetage complémentaire configuré pour coopérer avec un moyen d'encliquetage correspondant du manchon (10) pour assurer l'assemblage du corps de pompe sur la paroi intérieure du manchon (10), chaque moyen d'encliquetage complémentaire étant placé sur la surface externe du corps de pompe en étant distinct dudit au moins un élément d'assemblage.

15. Pompe selon la revendication 14, **caractérisée en ce que** ledit corps de pompe présente un joint périphérique placé en amont des moyens d'encliquetage pour assurer l'étanchéité de l'assemblage corps de pompe/manchon (10).

## Patentansprüche

1. Anordnung zum Einsetzen/Entfernen einer Herzpumpe (36) in eine bzw. von einer Hülse, die in einer Öffnung einer Ventrikelwand (33) befestigt ist, wobei die Anordnung ein Führungselement (30) mit einem distalen Ende, einem proximalen Ende und einem Lumen (31) umfasst, das sich zwischen dem distalen und dem proximalen Ende erstreckt und in diese einmündet, wobei die Herzpumpe (36) einen Pumpenkörper aufweist,
**dadurch gekennzeichnet, dass** der Pumpenkörper ein Montageelement umfasst, wobei die Anordnung ein Greifelement (37) umfasst, das dazu geeignet ist, in dem Lumen (31) zu gleiten, wobei das Greifelement (37) an seinem freien Ende einen zu dem Montageelement komplementären Montageabschnitt umfasst, der konfiguriert ist, um mit dem Montageelement zusammenzuwirken und das freie Ende mit dem Pumpenkörper zu verbinden, um das Greifen und das Bewegen der Herzpumpe (36) in dem Lumen (31) des Führungselements (30) bis zu der Hülse zu ermöglichen.

2. Anordnung nach Anspruch 1, **dadurch gekennzeichnet, dass das** Montageelement ein Hohlraum oder ein Vorsprung ist, der an oder in dem Pumpenkörper ausgebildet ist, wobei der komplementäre Montageabschnitt ein Kopf ist, dessen Form zu dem Hohlraum passt, bzw. eine Vertiefung ist, deren Form zu dem Vorsprung passt, um das freie Ende an dem Pumpenkörper zu montieren.

3. Anordnung nach Anspruch 1 oder 2, **dadurch gekennzeichnet, dass** das freie Ende des Greifelements (37) ein polygonales Profil aufweist, das zu einem Hohlprofil komplementär ist, welches an einem Ende des Körpers der Herzpumpe (36) so angeordnet ist, dass das freie Ende in das Hohlprofil eingeführt werden kann.

4. Anordnung nach einem der Ansprüche 1 bis 3, **dadurch gekennzeichnet, dass** der Pumpenkörper mindestens zwei Montageelemente umfasst, wobei das freie Ende des Greifelements (37) für jedes Montageelement einen zu dem entsprechenden Montageelement komplementären Montageabschnitt umfasst.

5. Anordnung nach einem der Ansprüche 1 bis 3, **dadurch gekennzeichnet, dass** der Pumpenkörper eine Öffnung aufweist, die in mindestens ein Gehäuse zum Aufnehmen/Halten des Pumpenkörpers mündet, wobei das freie Ende des Greifelements (37) einen rotationsbeweglichen Kopf umfasst, der ein oder mehrere Indexierungsreliefs aufweist, die durch Drehen des Kopfes eine entriegelte Position des Pumpenkörpers und des Endes des Greifelements (37) definieren, um deren Trennung zu ermöglichen, wobei der Kopf dazu in der Lage ist, frei durch die Öffnung zu gelangen, um in das Gehäuse einzutreten oder aus diesem auszutreten, und eine verriegelte Position definieren, in der das oder die Indexierungsreliefs in das Gehäuse zum Aufnehmen/Halten eingreifen, um eine mechanische Montage des Endes des Greifelements (37) und der Herzpumpe (36) sicherzustellen.

6. Anordnung nach einem der Ansprüche 1 bis 5, **dadurch gekennzeichnet, dass** das Greifelement (37) ein länglicher Körper von allgemein zylindrischer Form ist, so dass er im Lumen (31) des Führungselements (30) zur Drehung angetrieben werden kann.

7. Anordnung nach einem der Ansprüche 1 bis 6, **dadurch gekennzeichnet, dass** sie ein Dichtungselement umfasst, das innerhalb des Lumens (31) angeordnet ist, und vorzugsweise in seinem distalen Teil, wobei das Dichtungselement offen ist, wenn das freie Ende des Greifelements (37) dagegen gedrückt wird, um den Durchgang durch dieses Dichtungselement hindurch für das Greifelement (37) freizugeben.

8. Anordnung nach einem der Ansprüche 1 bis 7, **dadurch gekennzeichnet, dass** das distale Ende des Führungselements (30) eine Stabilisierungsvorrichtung für einen Teil des Herzens umfasst, wobei die Stabilisierungsvorrichtung einen aufgeweiteten Teil umfasst, der, vorzugsweise an seinem Ende mit größerem Durchmesser, eine Nut umfasst, die mindestens eine Saugöffnung aufweist, um ein Vakuum in der Nut zu erzeugen, wenn ein Teil der Ventrikelwand (33) mit der Stabilisierungsvorrichtung in Kontakt steht.

9. Anordnung nach einem der Ansprüche 1 bis 8, **gekennzeichnet dadurch, dass** sie ein anderes Element oder mehrere andere Elemente umfasst, das/die aus der Gruppe ausgewählt ist/sind, die umfasst: ein Perforationswerkzeug, ein Dilatatorwerkzeug, ein Führungskabel ,..., sowie Kombinationen dieser Elemente, wobei jedes der Elemente oder von deren Kombinationen dazu geeignet ist, in dem Lumen (31) zu gleiten.

10. Herzpumpe zur ventrikulären Unterstützung eines schlagenden Herzens, wobei die Herzpumpe (36) einen Pumpenkörper aufweist, **dadurch gekennzeichnet, dass** der Pumpenkörper mindestens ein Montageelement umfasst, das konfiguriert ist, um mit dem freien Ende des Greifelements (37) einer Anordnung gemäß einem der Ansprüche 1 bis 9 zum Einsetzen/Entfernen einer Herzpumpe (36) in eine bzw. von einer Hülse, die in einer Öffnung einer Ventrikelwand befestigt ist, zusammenzuwirken, um das Greifen und das Bewegen der Herzpumpe (36) im Lumen (31) des Führungselements (30) der Anordnung bis zur Hülse zu ermöglichen.

11. Pumpe nach Anspruch 10, **dadurch gekennzeichnet, dass** es sich um eine treibende Herzpumpe (36) handelt.

12. Pumpe nach Anspruch 10 oder 11, **dadurch gekennzeichnet, dass** die Hülse (10) einen inneren Kanal umfasst, und die Pumpe dazu bestimmt ist, in dem Kanal der Hülse aufgenommen zu sein/werden, wobei sich die Hülse (10) in einer axialen Richtung erstreckt und ein vorderes axiales Ende (14) und ein hinteres axiales Ende (13) umfasst, wobei der innere Kanal in das vordere und hintere axiale Ende mündet, wobei mindestens ein Teil der Innenwand der Hülse (10), die den inneren Kanal begrenzt, ein Gewinde aufweist, wobei der Pumpenkörper ein Gewinde an einem Teil seiner Außenfläche umfasst, das sich von dem mindestens einen Montageelement unterscheidet, wobei das Gewinde zu dem Gewinde des mindestens einen Teils der Innenwand der Hülse (10) komplementär ist, um deren Eingreifen sicherzustellen.

13. Pumpe nach Anspruch 12, **dadurch gekennzeichnet, dass** die Gewinde so konfiguriert sind, dass sie die Abdichtung der Anordnung Pumpenkörper/Hülse (10) sicherstellen.

14. Pumpe nach Anspruch 10 oder 11, **dadurch gekennzeichnet, dass** die Hülse (10) einen inneren Kanal umfasst und die Pumpe dazu bestimmt ist, in dem Kanal der Hülse aufgenommen zu sein/werden, wobei sich die Hülse (10) in einer axialen Richtung erstreckt und ein vorderes axiales Ende (14) und ein hinteres axiales Ende (13) umfasst, wobei der innere Kanal in das vordere und hintere axiale Ende mündet, wobei die Innenwand der Hülse (10), die den inneren Kanal begrenzt, mindestens zwei Rastmittel umfasst, wobei der Pumpenkörper für jedes Rastmittel der Hülse (10) ein komplementäres Rastmittel umfasst, das konfiguriert ist, um mit einem entsprechenden Rastmittel der Hülse (10) zusammenzuwirken, um die Montage des Pumpenkörpers an Innenwand der Hülse (10) sicherzustellen, wobei jedes komplementäre Rastmittel an der Außenfläche des Pumpenkörpers angeordnet ist, wobei es sich von dem mindestens einen Montageelement unterscheidet.

15. Pumpe nach Anspruch 14, **dadurch gekennzeichnet, dass** der Pumpenkörper eine Umfangsdichtung aufweist, die stromaufwärts von den Rastmitteln angeordnet ist, um die Anordnung Pumpenkörper/Hülse (10) abzudichten.

## Claims

1. Assembly for fitting/removing a heart pump (36) in a sleeve secured in an opening in a ventricular wall (33), said assembly comprising a guide element (30) with a distal end, a proximal end, and a lumen (31) extending between, and opening at, said distal and proximal ends, said heart pump (36) having a pump body,
**characterized in that**, with said pump body comprising an assembly element, said assembly comprises a gripping unit (37) which can slide in said lumen (31), said gripping unit (37) comprising at its free end an assembly part which is complementary with said assembly element, which part is configured to cooperate with said assembly element, and to join this free end to the pump body, in order to permit the gripping and displacement of said heart pump (36) in said lumen (31) of said guide element (30) of said assembly as far as said sleeve.

2. Assembly according to Claim 1, **characterized in that**, with said assembly element being a hollow, or a projection, provided in or on the pump body, said complementary assembly part is a head with a form paired with said hollow, or respectively a hollow with a form paired with said projection, for assembly of said free end on said pump body.

3. Assembly according to Claim 1 or 2, **characterized in that** the free end of said gripping unit (37) comprises a polygonal profile which is complementary with a hollow profile placed at an end of the heart pump body (36), such that this free end can be inserted in said hollow profile.

4. Assembly according to any one of Claims 1 to 3, **characterized in that** with said pump body comprising at least two assembly elements, said free end of said gripping unit (37) comprises for each assembly element an assembly part which is complementary with the corresponding assembly element.

5. Assembly according to any one of Claims 1 to 3, **characterized in that**, with said pump body comprising an orifice opening into at least one receptacle for receipt/retention of the pump body, the free end of said gripping unit (37) comprises a head which is maneuverable in rotation, which head has one or a plurality of indexing reliefs defining, by rotation of the head, an unlocked position of the pump body and of the end of the gripping unit (37), in order to permit their separation, said head being able to pass freely through said orifice in order to penetrate into, or exit from, said receptacle, and a locked position, in which the indexing relief(s) is/are engaged in the receipt/retention receptacle in order to provide mechanical assembly of said end of the gripping unit (37) and said heart pump (36).

6. Assembly according to any one of Claims 1 to 5, **characterized in that** said gripping unit (37) is an elongate body with a generally cylindrical form, such that it can be rotated in the lumen (31) of said guide element (30).

7. Assembly according to any one of Claims 1 to 6, **characterized in that** it comprises a sealing element placed inside said lumen (31), and preferably in its distal part, this sealing element being open when the free end of said gripping unit (37) is pressed against it, in order to open up the passage for said gripping unit (37) through this sealing element.

8. Assembly according to any one of Claims 1 to 7, **characterized in that** the distal end of said guide element (30) comprises a device for stabilization of part of the heart, said stabilization device comprising a widened part, which preferably comprises at its end with the widest diameter a groove which has at least one aspiration orifice for creation of a vacuum in said groove when a portion of the ventricular wall (33) is in contact with said stabilization device.

9. Assembly according to any one of Claims 1 to 8, **characterized in that** it comprises one or a plurality of other elements selected from the group comprising a perforation tool, a dilator tool, a guide wire, etc., and combinations of these elements, each of said elements or their combinations being able to slide in said lumen (31).

10. Heart pump for ventricular assistance for a beating heart, said heart pump (36) having a pump body, **characterized in that** said pump body comprises at least one assembly element which is configured to cooperate with the free end of the gripping unit (37) of an assembly for fitting/removing a heart pump (36) in a sleeve secured in an opening in a ventricular wall according to any one of Claims 1 to 9, in order to permit gripping and displacement of said heart pump (36) in the lumen (31) of the guide element (30) of said assembly as far as said sleeve.

11. Pump according to Claim 10, **characterized in that** it is a propulsive heart pump (36).

12. Pump according to Claim 10 or 11, **characterized in that**, with said sleeve (10) comprising an inner channel and said pump being designed to be received in the channel of said sleeve, said sleeve (10) extending in an axial direction and comprising a front axial end (14) and a rear axial end (13), said inner channel opening at the front and rear axial ends, at least part of the inner wall of said sleeve (10) delimiting said inner channel having a thread, said pump body comprises a thread on part of its outer surface which is distinct from said at least one assembly element, said thread being complementary with the thread of said at least part of the inner wall of the sleeve (10) in order to ensure their engagement.

13. Pump according to Claim 12, **characterized in that** said threads are configured to ensure the sealing of the pump body/sleeve assembly (10).

14. Pump according to Claim 10 or 11, **characterized in that**, with said sleeve (10) comprising an inner channel, and said pump being designed to be received in the channel of said sleeve, said sleeve (10) extending in an axial direction and comprising a front axial end (14) and a rear axial end (13), said inner channel opening at the front and rear axial ends, the inner wall of said sleeve (10) delimiting said inner channel comprising at least two means for snapping-in, said pump body comprises for each means for snapping-in of the sleeve (10) a complementary means for snapping-in, which is configured to cooperate with a corresponding means for snapping-in of the sleeve (10) in order to ensure the assembly of the pump body on the inner wall of the sleeve (10), each complementary means for snapping-in being placed on the outer surface of the pump body, whilst being distinct from said at least one assembly element.

15. Pump according to Claim 14, **characterized in that** said pump body has a peripheral seal placed upstream from the means for snapping-in, in order to ensure the sealing of the pump body/sleeve assembly (10).
